(19) Europäisches Patentamt

European Patent Office

Office européen des brevets

(11) **EP 1 522 536 A1**

(12) **EUROPEAN PATENT APPLICATION**
published in accordance with Art. 158(3) EPC

(43) Date of publication:
**13.04.2005 Bulletin 2005/15**

(51) Int Cl.⁷: **C07C 43/13**, C07C 41/06

(21) Application number: **03761767.7**

(22) Date of filing: **19.06.2003**

(86) International application number:
**PCT/JP2003/007771**

(87) International publication number:
**WO 2004/002932 (08.01.2004 Gazette 2004/02)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **27.06.2002 JP 2002188062**
**04.07.2002 JP 2002195905**

(71) Applicant: **ASAHI GLASS COMPANY LTD.**
**Tokyo 100-8405 (JP)**

(72) Inventor: **FURUKAWA, Yutaka;**
**c/o Asahi Glass Company, Limited**
**Yokohama-shi, Kanagawa 221-8755 (JP)**

(74) Representative: **Müller-Boré & Partner**
**Patentanwälte**
**Grafinger Strasse 2**
**81671 München (DE)**

(54) **FLUORINE-CONTAINING ALCOHOLS AND PROCESS FOR PRODUCTION THEREOF**

(57)     A novel fluorine-containing alcohol and a method for its production.

A fluorine-containing alcohol represented by $R^1CY^1HCY^2Y^3OQ^1OH$ or $HOQ^2OCZ^1Z^2CZ^3HR^2CZ^4HCZ^5Z^6OQ^2OH$ (wherein $R^1$ is a monovalent fluorine-containing organic group, etc., $R^2$ is a bivalent fluorine-containing organic group, etc., $Y^1$ to $Y^3$ and $Z^1$ to $Z^6$ are fluorine atoms, and $Q^1$ and $Q^2$ are alkylene groups, etc.). A method for producing a fluorine-containing alcohol having a group represented by $-CX^1HCX^2X^3OQOH$ (wherein $X^1$, $X^2$ and $X^3$ are fluorine atoms, and Q is an alkylene group, etc.), which comprises reacting a compound having a group represented by $-CX^1=CX^2X^3$ with a diol represented by HOQOH in the presence of an alkali metal compound.

**Description**

TECHNICAL FIELD

The present invention relates to a novel fluorine-containing alcohol and a method for its production.

BACKGROUND ART

As a fluorine-containing compound having a terminal hydroxyl group, $R^f(CF_2CF_2)_nCH_2CH_2OH$ ($R^f$ is a polyfluoroalkyl group) may be mentioned, and the fluorine-containing compound can be produced by the following method.
A method which comprises telomerizing $R^fI$ and $CF_2=CF_2$ into $R^f(CF_2CF_2)_nI$ (n is an integer of at least 1), adding it to ethylene and converting the resulting $R^f(CF_2CF_2)_nCH_2CH_2I$ to $R^f(CF_2CF_2)_nCH_2CH_2OH$ by using a solid acid catalyst carrying an alkali metal (JP-A-2000-79345).
However, because $R^f(CF_2CF_2)_nI$ produced by telomerization reaction is a compound having a distribution in respect of the number of carbon atoms (i.e., n in the above chemical formula), $R^f(CF_2CF_2)_nCH_2CH_2OH$ derived from the compound is also a fluorine-containing compound having a distribution in respect of the number of carbon atoms. Accordingly, separation is required after the reaction in order to obtain a fluorine-containing compound having a terminal hydroxyl group and a specific number of carbon atoms.
The present inventors found that it is necessary to use a diol as a starting material for the reaction in order to solve the problem of the distribution in respect of the number of carbon atoms in the telomerization reaction, and found a novel compound having a terminal hydroxyl group based on the knowledge. Namely, the object of the present invention is to provide a novel fluorine-containing alcohol and a method for its production.

DISCLOSURE OF THE INVENTION

The present invention provides a fluorine-containing alcohol represented by the following formula 1 (compound 1) or the following formula 2 (compound 2):

$$R^1CY^1HCY^2Y^3OQ^1OH \qquad\qquad \text{Formula 1}$$

$$HOQ^2OCZ^1Z^2CZ^3HR^2CZ^4HCZ^5Z^6OQ^3OH \qquad\qquad \text{Formula 2}$$

wherein the symbols in the formulae (1) and (2) have the following meanings:

$R^1$: a monovalent organic group, a halogen atom or a hydrogen atom;
$R^2$: a bivalent organic group;
$Y^1$, $Y^2$ and $Y^3$: independently hydrogen atoms or fluorine atoms provided that when $R^1$ is not a fluorine atom, at least one of $Y^1$, $Y^2$ and $Y^3$ is a fluorine atom;
$Z^1$, $Z^2$, $Z^3$, $Z^4$, $Z^5$ and $Z^6$: independently hydrogen atoms or fluorine atoms provided that at least one of $Z^1$, $Z^2$ and $Z^3$ is a fluorine atom, and at least one of $Z^4$, $Z^5$ and $Z^6$ is a fluorine atom;
$Q^1$, $Q^2$ and $Q^3$: independently bivalent organic groups.

Further, the present invention provides a method for producing a fluorine-containing alcohol (compound 5) having a group represented by the following formula (5), which comprises reacting a compound (compound 3) having a group represented by the following formula (3) and a diol (compound 4) represented by the following formula (4), in the presence of an alkali metal compound:

$$-CX^1=CX^2X^3 \qquad\qquad \text{Formula 3}$$

$$HOQOH \qquad\qquad \text{Formula 4}$$

$$-CX^1HCX^2X^3OQOH \qquad\qquad \text{Formula 5}$$

provided that the symbols in the formulae (3), (4) and (5) have the following meanings:

$X^1$, $X^2$ and $X^3$: independently hydrogen atoms or fluorine atoms provided that at least one of $X^1$, $X^2$ and $X^3$ is a fluorine atom;
Q: a bivalent organic group.

## BEST MODE FOR CARRYING OUT THE INVENTION

In compound 1, $R^1$ is a monovalent organic group, a halogen atom or a hydrogen atom. $R^1$ is preferably a monovalent hydrocarbon group or a monovalent halogenated hydrocarbon group, particularly preferably a monovalent halogenated hydrocarbon group containing an etheric oxygen atom. $R^1$ may be linear, branched or cyclic.

Especially, $R^1$ is preferably a fluorohydrocarbon group, particularly preferably a perfluorohydrocarbon group having all the hydrogen atoms of a hydrocarbon group substituted by fluorine atoms, and most preferably a perfluorohydrocarbon group containing an etheric oxygen atom which, if any, is preferably present at a terminal portion.

In the compound 1, $Y^1$, $Y^2$ and $Y^3$ are preferably fluorine atoms, and it is preferred that all of them are fluorine atoms. Further, $Q^1$ is preferably a bivalent organic group, particularly preferably an alkylene group. Further, $Q^1$ is preferably a group represented by $-(CH_2)_t-$ (t is an integer of at least 1), and t is preferably from 2 to 12, particularly preferably from 2 to 6.

The compound 1 is preferably a compound (compound 6) represented by $R'CFHCF_2O(CH_2)_mOH$ (wherein R' is a monovalent fluorine-containing $C_{1-16}$ organic group containing an etheric oxygen atom, and m is an integer of from 1 to 12) because of ease of synthesis. In the compound 6, R' may be linear or branched, and if branched, preferably has a branch preferably represented by $(CF_3)_2CF-$ at an end.

Especially, R' is preferably a fluorinated hydrocarbon group, particularly preferably a perfluorohydrocarbon group having all the hydrogen atoms of a hydrocarbon group substituted by fluorine atoms, most preferably a perfluorohydrocarbon group containing an etheric oxygen atom which, if any, is preferably present at a terminal portion.

Specific examples of the compound 1 are the following compounds.

$$H(CF_2)_2O(CH_2)_2OH,$$

$$H(CF_2)_2O(CH_2)_3OH,$$

$$H(CF_2)_2O(CH_2)_4OH,$$

$$H(CF_2)_2O(CH_2)_6OH,$$

$$CF_3CFHCF_2O(CH_2)_2OH,$$

$$CF_3CFHCF_2O(CH_2)_3OH,$$

$$CF_3CFHCF_2O(CH_2)_4OH,$$

$$CF_3CFHCF_2O(CH_2)_6OH,$$

$$C_2F_5CFHCF_2O(CH_2)_2OH.$$

$$C_2F_5CFHCF_2O(CH_2)_3OH,$$

$C_2F_5CFHCF_2O(CH_2)_4OH,$

$C_2F_5CFHCF_2O(CH_2)_6OH,$

$C_3F_7CFHCF_2O(CH_2)_2OH,$

$C_3F_7CFHCF_2O(CH_2)_3OH,$

$C_3F_7CFHCF_2O(CH_2)_4OH,$

$C_3F_7CFHCF_2O(CH_2)_6OH,$

$C_5F_{11}CFHCF_2O(CH_2)_2OH,$

$C_5F_{11}CFHCF_2O(CH_2)_3OH,$

$C_5F_{11}CFHCF_2O(CH_2)_4OH,$

$C_5F_{11}CFHCF_2O(CH_2)_6OH,$

$C_7F_{15}CFHCF_2O(CH_2)_2OH,$

$C_7F_{15}CFHCF_2O(CH_2)_3OH,$

$C_7F_{15}CFHCF_2O(CH_2)_4OH,$

$C_7F_{15}CFHCF_2O(CH_2)_6OH.$

$C_3F_7OCFHCF_2O(CH_2)_2OH,$

$C_3F_7OCFHCF_2O(CH_2)_3OH,$

$C_3F_7OCFHCF_2O(CH_2)_4OH,$

$C_3F_7OCFHCF_2O(CH_2)_6OH,$

$C_4F_9OCFHCF_2OCH_2CH_2OH,$

$C_4F_9OCFHCF_2O(CH_2)_3OH,$

$C_4F_9OCFHCF_2O(CH_2)_4OH,$

$C_4F_9OCFHCF_2O(CH_2)_6OH,$

$C_5F_{11}OCFHCF_2O(CH_2)_2OH,$

$C_5F_{11}OCFHCF_2O(CH_2)_3OH,$

$C_5F_{11}OCFHCF_2O(CH_2)_4OH,$

$C_5F_{11}OCFHCF_2O(CH_2)_6OH,$

$C_6F_{13}OCFHCF_2O(CH_2)_2OH,$

$C_6F_{13}OCFHCF_2O(CH_2)_3OH,$

$C_6F_{13}OCFHCF_2O(CH_2)_4OH,$

$C_6F_{13}OCFHCF_2O(CH_2)_6OH.$

$(CF_3)_2CFCF_2CFHCF_2O(CH_2)_2OH,$

$(CF_3)_2CFCF_2CFHCF_2O(CH_2)_3OH,$

$(CF_3)_2CFCF_2CFHCF_2O(CH_2)_4OH,$

$(CF_3)_2CFCF_2CFHCF_2O(CH_2)_6OH,$

$F[CF(CF_3)CF_2O]CFHCF_2O(CH_2)_2OH,$

$F[CF(CF_3)CF_2O]CFHCF_2O(CH_2)_4OH,$

$F[CF(CF_3)CF_2O]_2CFHCF_2O(CH_2)_2OH,$

$F[CF(CF_3)CF_2O]_2CFHCF_2O(CH_2)_4OH,$

$$F[CF(CF_3)CF_2O]_3CFHCF_2O(CH_2)_2OH,$$

and

$$F[CF(CF_3)CF_2O]_3CFHCF_2O(CH_2)_4OH.$$

In the compound 2, $R^2$ is preferably a bivalent hydrocarbon group or a bivalent halogenated hydrocarbon group, particularly preferably a bivalent halogenated hydrocarbon group containing an etheric oxygen atom. $R^2$ may be linear, branched or cyclic.

Especially, $R^2$ is preferably a fluorinated hydrocarbon group, more preferably a perfluorohydrocarbon group having all the hydrogen atoms of a hydrocarbon group substituted by fluorine atoms, most preferably a perfluorohydrocarbon group containing an etheric oxygen atom which, if any, is preferably present at a terminal portion.

In the compound 2, $Z^1$, $Z^2$, $Z^3$, $Z^4$, $Z^5$ and $Z^6$ are preferably fluorine atoms, and it is preferred that all of them are fluorine atoms. Further, $Q^2$ and $Q^3$ are preferably the same as mentioned for $Q^1$ in the compound 1.

The compound 2 is preferably a compound (compound 7) represented by $R''[CFHCF_2O(CH_2)_qOH]_2$ (wherein $R''$ is a bivalent fluorine-containing $C_{1-16}$ organic group containing an etheric oxygen atom, and q is an integer of from 1 to 12) because of ease of synthesis. In the compound 7, $R''$ be linear or branched, and if branched, preferably has a branch preferably represented by $(CF_3)_2CF-$ at an end.

Especially, $R''$ is preferably a fluorinated hydrocarbon group, more preferably a perfluorohydrocarbon group having all the hydrogen atoms of a hydrocarbon group substituted by fluorine atoms, most preferably a perfluorohydrocarbon group containing an etheric oxygen atom which, if any, is preferably present at a terminal portion.

Specific examples of the compound 2 are the following compounds.

$$HO(CH_2)_2OCF_2CFH(CF_2)_2CFHCF_2O(CH_2)_2OH,$$

$$HO(CH_2)_3OCF_2CFH(CF_2)_2CFHCF_2O(CH_2)_3OH,$$

$$HO(CH_2)_4OCF_2CFH(CF_2)_2CFHCF_2O(CH_2)_4OH,$$

$$HO(CH_2)_6OCF_2CFH(CF_2)_2CFHCF_2O(CH_2)_6OH,$$

$$HOCH_2CH_2OCF_2CFH(CF_2)_3CFHCF_2OCH_2CH_2OH,$$

$$HO(CH_2C)_3OCF_2CFH(CF_2)_3CFHCF_2O(CH_2)_3OH,$$

$$HO(CH_2)_4OCF_2CFH(CF_2)_3CFHCF_2O(CH_2)_4OH,$$

$$HO(CH_2)_6OCF_2CFH(CF_2)_3CFHCF_2O(CH_2)_6OH.$$

$$HO(CH_2)_2OCF_2CFHO(CF_2)_2OCFHCF_2O(CH_2)_2OH,$$

$$HO(CH_2)_3OCF_2CFHO(CF_2)_2OCFHCF_2O(CH_2)_3OH,$$

$$HO(CH_2)_4OCF_2CFHO(CF_2)_2OCFHCF_2O(CH_2)_4OH,$$

$$HO(CH_2)_6OCF_2CFHO(CF_2)_2OCFHCF_2O(CH_2)_6OH,$$

$$HO(CH_2)_2OCF_2CFHO(CF_2)_3OCFHCF_2O(CH_2)_2OH,$$

$$HO(CH_2)_3OCF_2CFHO(CF_2)_3OCFHCF_2O(CH_2)_3OH,$$

$$HO(CH_2)_4OCF_2CFHO(CF_2)_3OCFHCF_2O(CH_2)_4OH,$$

$$HO(CH_2)_6OCF_2CFHO(CF_2)_3OCFHCF_2O(CH_2)_6OH,$$

$$HO(CH_2)_2OCF_2CFH[OCF_2CF(CF_3)]OCF_2(CF_2)_4CF_2O[CF(CF_3)CF_2O]C$$

$$FHCF_2O(CH_2)_2OH.$$

$$HO(CH_2)_3OCF_2CFH[OCF_2CF(CF_3)]OCF_2(CF_2)_4CF_2O[CF(CF_3)CF_2O]C$$

$$FHCF_2O(CH_2)_3OH,$$

$$HO(CH_2)_4OCF_2CFH[OCF_2CF(CF_3)]OCF_2(CF_2)_4CF_2O[CF(CF_3)CF_2O]C$$

$$FHCF_2O(CH_2)_4OH$$

and

$$HO(CH_2)_6OCF_2CFH[OCF_2CF(CF_3)]OCF_2(CF_2)_4CF_2O[CF(CF_3)CF_2O]C$$

$$FHCF_2O(CH_2)_6OH.$$

In the compound 3, $X^1$, $X^2$ and $X^3$ are preferably fluorine atoms, and it is preferred that all of them are fluorine atoms. The compound 3 can be prepared by the method disclosed in J. Am. Chem. Soc, 75, 4525 (1953) or the like.

The compound 3 is preferably a compound (compound 8) represented by the following formula 8.

$$R[CX^1=CX^2X^3]_p \hspace{4cm} \text{Formula 8}$$

R: a p-valent organic group;
p: an integer of from 1 to 4;
$X^1$, $X^2$ and $X^3$: independently hydrogen atoms or fluorine atoms provided that at least one of $X^1$, $X^2$ and $X^3$ is a fluorine atom.

In the compound 8, R is preferably the same as mentioned for $R^1$ in the compound 1 or for $R^2$ in the compound 2. $X^1$, $X^2$ and $X^3$ are preferably a fluorine atom, and it is preferred that all of them are fluorine atoms. p is preferably 1 or 2.

Specific examples of the compound 3 are the following compounds.

$CF_2=CF_2$, $CF_3CF=CF_2$, $C_2F_5CF=CF_2$, $C_3F_7CF=CF_2$, $C_5F_{11}CF=CF_2$,
$C_7F_{15}CF=CF_2$, $C_3F_7OCF=CF_2$, $C_4F_9OCF=CF_2$, $C_5F_{11}OCF=CF_2$,
$C_6F_{13}OCF=CF_2$, $(CF_3)_2CFCF_2CF=CF_2$, $F[CF(CF_3)CF_2O]CF=CF_2$,
$F[CF(CF_3)CF_2O]_2CF=CF_2$, $F[CF(CF_3)CF_2O]_3CF=CF_2$.
$CF_2=CF(CF_2)_2CF=CF_2$, $CF_2=CF(CF_2)_3CF=CF_2$,
$CF_2=CFO(CF_2)_2OCF=CF_2$, $CF_2=CFO(CF_2)_3OCF=CF_2$ and

$$CF_2=CF[OCF_2CF(CF_3)]OCF_2(CF_2)_4CF_2O[CF(CF_3)CF_2O]CF=CF_2.$$

In the compound 4, Q is preferably the same as mentioned for $Q^1$ in the compound 1.

As specific examples of the compound 4, $HO(CH_2)_2OH$, $HO(CH_2)_3OH$, $HO(CH_2)_4OH$ and $HO(CH_2)_6OH$ are preferably mentioned.

In the compound 5, $X^1$, $X^2$, $X^3$ and Q are preferably the same as those in the compound 3 or 4.

The compound 5 is preferably a compound (compound 9) represented by the following formula 9.

$$R[CX^1HCX^2X^3OQOH]_p \qquad \text{Formula 9}$$

R: a p-valent organic group;

$X^1$, $X^2$ and $X^3$: independently hydrogen atoms or fluorine atoms provided that at least one of $X^1$, $X^2$ and $X^3$ is a fluorine atom;

Q: a bivalent organic group;

P: an integer of from 1 to 4.

In the compound 9, R, $X^1$, $X^2$, $X^3$, Q and p are preferably the same as those in the compound 4 or the compound 8. The compound 5 is preferably the compound 1 or the compound 2.

The alkali metal compound to be used in the reaction of the compound 3 and the compound 4 is preferably an alkali metal, an alkali metal hydride, an alkali metal hydroxide, an alkali metal amide or the like. Specifically, an alkali metal such as Na, K or Cs, an alkali metal hydroxide such as NaOH or KOH, an alkali metal hydride such as NaH or KH, or an alkali metal amide such as $NaNH_2$, $KNH_2$ is preferably mentioned.

In the reaction, the alkali metal compound is preferably used in an amount of from 0.01 to 1.0 mol, particularly preferably in an amount of from 0.05 to 0.5 mol in view of the reaction rate, based on 1 mol of the compound 3, though there is no particular restriction. The usage range is preferred because the reaction proceeds at an appropriate reaction rate with little production of by-products.

In the reaction, the compound 4 is preferably used in an amount of from 1 to 5 mols, particularly preferably from 2 to 4 mols based on 1 mol of the compound 3 for production of the compound having a group represented by the formula 5, namely the compound 1, and is preferably used in an amount of from 2 to 10 mols, particularly preferably from 4 to 8 mols based on 1 mol of the compound 3 for production of the compound having two groups represented by the formula 5, namely the compound 2. The reaction is preferably carried out in the range because the compound 3 is likely to react with only one hydroxyl group in the compound 4 to give the product.

Further, the temperature for the reaction is preferably from 0 to 150°C, particularly preferably from 40 to 120°C. The reaction is preferably carried out within this temperature range because the reaction proceeds at an appropriate reaction rate without homopolymerization of the compound 3.

Further, in the reaction, a solvent may or may not be used, but it is preferably used. The solvent is preferably a solvent which dissolves the compound 4, a solvent which dissolves the compound 5, or a solvent which is practically inert in the reaction. The solvent is preferably an ether, a nitrile compound or the like, and specifically, it is preferably diethyl ether, glyme, dioxane, tetrahydrofuran, acetonitrile or propionitrile, particularly preferably dioxane, tetrahydrofuran or acetonitrile.

The solvent is preferably used in such an amount that the compound 5 as the product accounts for from 1 to 60 mass%, particularly from 3 to 50 mass% in view of the reaction rate and the productivity, though there is no particular restriction.

Further, the compound 3 tends to polymerize at high pressure, and therefore in order to prevent the polymerization, a polymerization inhibitor is preferably used in the reaction. The polymerization inhibitor may be put in the reaction system before or with the starting materials. The polymerization inhibitor is preferably limonene, pinene, cymene, terpinene or the like, though there is no particular restriction.

In the present invention, the compound 5 is presumed to be produced by the following mechanism.

Namely, the alkali metal compound converts the diol (compound 4) to an alkoxide compound, then the alkoxide compound is added to the compound 3, and the metal portion is replaced by a hydrogen atom to produce the compound 5. The use of a diol allows production of a fluorine-containing alcohol because one hydroxyl group remains after the other hydroxyl group is reacted.

According to the present invention, a novel fluorine-containing alcohol can be produced. The fluorine-containing alcohol is useful as an intermediate of cleaning agents or various compounds. For example, a fluorine-containing acrylate obtained by reacting the compound 1 with acrylic acid is useful as a starting material for a UV curable resin or a water and oil repellent. Further, when used as a comonomer component for a condensation resin such as a urethane, the compound 2 can alter the surface properties of the resin.

Further, the compound 3 as the starting material for the fluorine-containing alcohol of the present invention can be prepared by the direct fluorination disclosed in Adv. Synth. Catal. 2001, 343, No. 2, which allows a choice of the structure of the starting material and therefore can give fluorine-containing alcohols in various structures.

EXAMPLES

Now, the present invention will be described in detail with reference to Examples, but it should be understood that the present invention is by no means restricted thereto.

EXAMPLE 1

30 g of 1,4-dioxane, 10 g of $C_3F_7OCF=CF_2$, 4.67 g of $HO(CH_2)_2OH$ and 0.55 g of KOH were sealed in a stainless steel reaction vessel having a capacity of 50 mL, then reacted at 70°C for 8 hours with stirring, and after addition of 50 mL of water, allowed to separate into two layers. Then, the organic layer was distilled to obtain 10.9 g of $CF_3CF_2CF_2OCFHCF_2OCH_2CH_2OH$ and $C_3F_7OCFHCF_2OCH_2CH_2OCF_2CFHOC_3F_7$ as the products in the ratio of 75:25 (molar ratio).
The results of analysis of $CF_3CF_2CF_2OCFHCF_2OCH_2CH_2OH$ are shown below.

> IR (neat): 3395, 1342, 1236, 1199, 1153, 1102, 985 cm$^{-1}$,
> $^1$H-NMR(CDCl$_3$)δ: 1.84 (t, J=6.3 Hz, 3H, OH), 3.86 - 3.94 (m, 2H, CH$_2$OH), 4.15 (t, J=4.6 Hz, 2H, CF$_2$OCH$_2$), 5.95 (d, t, 53.5 Hz, 2.9 Hz, 1H, CFHCF$_2$),
> $^{19}$F-NMR(CDCl$_3$)δ: -81.4 (t, J=6.4 Hz, 3F, CF$_3$), -84.88 and -86.99 (ABquartet, J=145.1 Hz, 2F, CF$_2$OCFH), -89.00 and -89.84 (ABquartet, J=144.0 Hz, 2F, CF$_2$OCH$_2$), -129.50 - -129.60 (m, 2F, CF$_2$CF$_3$), -144.0 (d, quintet, J=53.5 Hz, 6.4 Hz, 1F, CFH).

EXAMPLE 2

30 g of 1,4-dioxane, 10 g of $C_3F_7OCF=CF_2$, 13.55 g of $HO(CH_2)_4OH$ and 0.55 g of KOH were sealed in a stainless steel reaction vessel having a capacity of 50 mL, reacted at 70°C for 8 hours with stirring, and after addition of 50 mL of water, allowed to separate into two layers. Then, the organic layer was distilled to obtain 12.8 g of $CF_3CF_2CF_2OCFHCF_2O(CH_2)_4OH$ and $C_3F_7OCFHCF_2O(CH_2)_4OCF_2CFHOC_3F_7$ as the products in the ratio of 94:6 (molar ratio).
The results of analysis of $CF_3CF_2CF_2OCFHCF_2O(CH_2)_4OH$ are shown below.

> IR (neat): 3349, 2951, 1341, 1237, 1199, 1153, 987 cm$^{-1}$,
> $^1$H-NMR(CDCl$_3$)δ: 1.39 (s, 1H, OH), 1.60 - 1.73 (m, 2H, CH$_2$CH$_2$OH), 1.73 - 1.86 (m, 2H, CF$_2$OCH$_2$CH$_2$), 3.63 - 3.76 (m, 2H, CH$_2$OH), 4.03 (t, J=6.3 Hz, 2H, CF$_2$OCH$_2$), 5.86 (d, t, 53.7 Hz, 2.8 Hz, 1H, CFHCF$_2$),
> $^{19}$F-NMR(CDCl$_3$)δ: -81.4 (t, J=7.5 Hz, 3F, CF$_3$), -84.86 and -86.96 (ABquartet, J=145.0 Hz, 2F, CF$_2$OCFH), -89.30 and -89.94 (ABquartet, J=144.0 Hz, 2F, CF$_2$OCH$_2$), -129.5 - -129.6 (m, 2F, CF$_2$CF$_3$), -144.2 (d, quintet, J=53.7 Hz, 8.6 Hz, 1F, CFH).

EXAMPLE 3

30 g of 1,4-dioxane, 10 g of $C_3F_7OCF=CF_2$, 5.72g of $HOCH_2CH_2CH_2OH$ and 0.55 g of KOH were sealed in a stainless steel reaction vessel having a capacity of 50 mL, reacted at 70°C for 8 hours with stirring, and after addition of 50 mL of water, allowed to separate into two layers. Then, the organic layer was distilled to obtain 11.6 g of $CF_3CF_2CF_2OCFHCF_2OCH_2CH_2CH_2OH$ and $C_3F_7OCFHCF_2OCH_2CH_2CH_2OCF_2CFHOC_3F_7$ as the products in the ratio of 89:11 (molar ratio).
The results of analysis of $CF_3CF_2CF_2OCFHCF_2OCH_2CH_2CH_2OH$ are shown below.

> IR(neat): 3356, 1342, 1236, 1199, 1153, 1098, 987 cm$^{-1}$,
> $^1$H-NMR(CDCl$_3$)δ: 1.58 (s, 1H, OH), 1.93 (quintet, J=6.1 Hz, 2H, CH$_2$CH$_2$CH$_2$), 3.76 (t, J=6.0 Hz, 2H, CH$_2$OH), 4.14 (t, J=6.1 Hz, 2H, CF$_2$OCH$_2$), 5.87 (d, t, 53.5 Hz, 2.9 Hz, 1H, CFHCF$_2$),
> $^{19}$F-NMR (CDCl$_3$)δ: -81.4 (t, J=7.5 Hz, 3F, CF$_3$), -84.88 and -87.02 (ABquartet, J=148.0 Hz, 2F, CF$_2$OCFH), -89.36 and -90.05 (ABquartet, J=146.1 Hz, 2F, CF$_2$OCH$_2$), -129.54 - -129.64 (m, 2F, CF$_2$CF$_3$), -144.2 (d, quintet, J=53.5 Hz, 8.1 Hz, 1F, CFH).

EXAMPLE 4

30 g of 1,4-dioxane, 10 g of $C_3F_7OCF=CF_2$, 8.88 g of $HO(CH_2)_6OH$ and 0.55 g of KOH were sealed in a stainless steel reaction vessel having a capacity of 50 mL, reacted at 70°C for 8 hours with stirring, and after addition of 50 mL of water, allowed to separate into two layers. Then, the organic layer was distilled to obtain 13.0 g of $CF_3CF_2CF_2OCFHCF_2O(CH_2)_6OH$ and $C_3F_7OCFHCF_2O(CH_2)_6OCF_2CFHOC_3F_7$ as the products in the ratio of 87:13 (molar ratio).
The results of analysis of $CF_3CF_2CF_2OCFHCF_2O(CH_2)_6OH$ are shown below.

IR(neat): 3351, 2942, 1341, 1237, 1199, 1154, 1092, 988 cm$^{-1}$,
$^1$H-NMR(CDCl$_3$)δ: 1.32 - 1.48 (5H, OH, $CH_2CH_2CH_2CH_2CH_2CH_2$), 1.52 - 1.75 (m, 4H, $CH_2CH_2CH_2CH_2CH_2CH_2$), 3.65 (t, J=6.5 Hz, 2H, $CH_2OH$), 3.98 (t, J=6.5 Hz, 2H, $CF_2OCH_2$), 5.85 (d, t, 54.1 Hz, 2.9 Hz, 1H, $CFHCF_2$),
$^{19}$F-NMR (CDCl$_3$)δ: -81.3 (t, J=6.4 Hz, 3F, $CF_3$), -84.71 and -86.86 (ABquartet, J=146.1 Hz, 2F, $CF_2OCFH$), -89.06 and -89.85 (ABquartet, J=144.0 Hz, 2F, $CF_2OCH_2$), -129.38 - 129.50 (m, 2F, $CF_2CF_3$), -144.1 (d, t, J=54.1 Hz, 1F, CFH).

EXAMPLE 5

30 g of 1,4-dioxane, 10 g of $CF_2=CFO(CF_2)_2OCF=CF_2$, 12.26 g of $HO(CH_2)_4OH$ and 0.55 g of KOH were sealed in a stainless steel reaction vessel having a capacity of 50 mL, reacted at 70°C for 8 hours with stirring, and after addition of 50 mL of water, allowed to separate into two layers. Then, the organic layer was distilled to obtain 13.2 g of $HO(CH_2)_4OCF_2HCFO(CF_2)_2OCFHCF_2O(CH_2)_4OH$ as the product.
The results of analysis of $HO(CH_2)_4OCF_2HCFO(CF_2)_2OCFHCF_2O(CH_2)_4OH$ are shown below.

IR(neat): 3349, 2951, 1341, 1237, 1199, 1153, 987 cm$^{-1}$,
$^1$H-NMR(CDCl$_3$)δ: 1.39 (s, 2H, OH), 1.60 - 1.73 (m, 4H, $CH_2CH_2OH$), 1.73 - 1.86 (m, 4H, $CF_2OCH_2CH_2$), 3.63 - 3.76 (m, 4H, $CH_2OH$), 4.03 (t, J=6.3 Hz, 4H, $CF_2OCH_2$), 5.86 (d, t, 53.7 Hz, 2.8 Hz, 2H, $CFHCF_2$),
$^{19}$F-NMR (CDCl$_3$)δ: -84.86 and -86.96 (ABquartet, J=145.0 Hz, 4F, $CF_2OCFH$), -89.30 and -89.94 (ABquartet, J=144.0 Hz, 4F, $CF_2OCH_2$), -144.2 (d, quintet, J=53.7 Hz, 8.6 Hz, 2F, CFH).

**Claims**

1. A fluorine-containing alcohol represented by the following formula (1) or (2):

$$R^1CY^1HCY^2Y^3OQ^1OH \hspace{3cm} \text{Formula 1}$$

$$HOQ^2OCZ^1Z^2CZ^3HR^2CZ^4HCZ^5Z^6OQ^3OH \hspace{2cm} \text{Formula 2}$$

wherein the symbols in the formulae (1) and (2) have the following meanings:

$R^1$: a monovalent organic group, a halogen atom or a hydrogen atom;
$R^2$: a bivalent organic group;
$Y^1$, $Y^2$ and $Y^3$: independently hydrogen atoms or fluorine atoms provided that when $R^1$ is not a fluorine atom, at least one of $Y^1$, $Y^2$ and $Y^3$ is a fluorine atom;
$Z^1$, $Z^2$, $Z^3$, $Z^4$, $Z^5$ and $Z^6$: independently hydrogen atoms or fluorine atoms provided that at least one of $Z^1$, $Z^2$ and $Z^3$ is a fluorine atom, and at least one of $Z^4$, $Z^5$ and $Z^6$ is a fluorine atom;
$Q^1$, $Q^2$ and $Q^3$: independently bivalent organic groups.

2. A method for producing a fluorine-containing alcohol having a group represented by the following formula (5), which comprises reacting a compound having a group represented by the following formula (3) and a diol represented by the following formula (4), in the presence of an alkali metal compound:

$$-CX^1=CX^2X^3 \hspace{3cm} \text{Formula 3}$$

$$HOQOH \qquad \text{Formula 4}$$

$$-CX^1HCX^2X^3OQOH \qquad \text{Formula 5}$$

wherein the symbols in the formulae (3), (4) and (5) have the following meanings:

$X^1$, $X^2$ and $X^3$: independently hydrogen atoms or fluorine atoms provided that at least one of $X^1$, $X^2$ and $X^3$ is a fluorine atom;

Q: a bivalent organic group.

3. The fluorine-containing alcohol according to Claim 1, wherein $R^1$ in the formula (1) is a monovalent perfluorohydrocarbon group containing an etheric oxygen atom, and $R^2$ in the formula (2) is a bivalent perfluorohydrocarbon group containing an etheric oxygen atom.

4. The fluorine-containing alcohol according to Claim 1, wherein $Q^1$ in the formula (1), and $Q^2$ and $Q^3$ in the formula (2) are $-(CH_2)_t-$ (t is an integer of at least 1).

5. The method for producing a fluorine-containing alcohol according to Claim 2, wherein Q in the formula (5) is $-(CH_2)_t-$ (t is an integer of at least 1).

## INTERNATIONAL SEARCH REPORT

| International application No. |
| --- |
| PCT/JP03/07771 |

**A. CLASSIFICATION OF SUBJECT MATTER**
Int.Cl⁷ C07C43/13, 41/06

According to International Patent Classification (IPC) or to both national classification and IPC

**B. FIELDS SEARCHED**

Minimum documentation searched (classification system followed by classification symbols)
Int.Cl⁷ C07C43/13, 41/06

Documentation searched other than minimum documentation to the extent that such documents are included in the fields searched

Electronic data base consulted during the international search (name of data base and, where practicable, search terms used)
CA(STN), CAOLD(STN), REGISTRY(STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | CIRKVA V. et al., 'Radical additions to fluoroolefins.', J.Fluorine.Chem. 1996, Vol.80, pages 135 to 144 | 1-5<br>3 |
| X<br>Y | US 2631975 A (American Viscose Corp.), 17 March, 1953 (17.03.53), Claims; example I (Family: none) | 1,2,4,5<br>2,3,5 |
| X<br>Y | US 2409274 A (E.I. du Pont de Nemours & Co.), 15 October, 1946 (15.10.46), Claims; example IV; Column 6, lines 21 to 39 (Family: none) | 1,2,4,5<br>3 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>05 September, 2003 (05.09.03) | Date of mailing of the international search report<br>30 September, 2003 (30.09.03) |
| --- | --- |
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

| International application No. |
|---|
| PCT/JP03/07771 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | BREY M.L. et al., 'The Preparation and Properties of Some Vinyl and Glycidyl Fluoroethers', J.Am. Chem.Soc., 1957, Vol.79, pages 6533 to 6536 | 1,2,4,5<br>3 |
| X<br>Y | PARK J.D. et al., 'Preparation of 3-(1,1,2-Trifluoro-2-chloroethoxy) propanol and Some of Its Derivatives', J.Org.Chem., 1958, Vol.23, pages 1394 to 1395 | 1,2,4,5<br>3 |
| X<br>Y<br>A | JP 63-192732 A  (Canon Inc.),<br>10 August, 1988 (10.08.88),<br>Claims<br>(Family: none) | 1,4<br>2,5<br>3 |
| X<br>Y<br>A | JP 53-113028 A  (Kansai Paint Co., Ltd.),<br>03 October, 1978 (03.10.78),<br>Example 1<br>(Family: none) | 1,4<br>2,5<br>3 |
| X<br>A | JP 1-226844 A  (Tokuyama Soda Co., Ltd.),<br>11 September, 1989 (11.09.89),<br>Example 7<br>(Family: none) | 1<br>2-5 |

Form PCT/ISA/210 (continuation of second sheet) (July 1998)